# EUROPEAN PATENT SPECIFICATION

(11) **EP 2 983 741 B1**
(45) Date of publication and mention of the grant of the patent: **03.06.2020**
(21) Application number: 14716788.6
(22) Date of filing: 08.04.2014
(51) Int. Cl.: A61M 5/20, A61M 5/315, A61M 5/31

(54) **PEN-TYPE DRUG INJECTION DEVICE AND DOSE SETTING LIMITER MECHANISM THEREFOR**
STIFTARTIGER ARZNEIMITTELINJEKTOR MIT BEGRENZUNGSMECHANISMUS FÜR DESSEN DOSISEINSTELLUNG
DISPOSITIF D'INJECTION DE MÉDICAMENTS DU TYPE STYLO AVEC MÉCANISME LIMITEUR DE DOSAGE

(30) Priority: 10.04.2013 EP 13163062
(43) Date of publication of application: 17.02.2016
(73) Proprietor: SANOFI, 75008 Paris (FR)
(72) Inventor: HIGGINS, Daniel David, Bristol BS8 3LB (GB); PLUMPTRE, David Aubrey, Droitwich Spa Worcestershire WR9 7RQ (GB); VEASEY, Robert, Leamington Spa Warwickshire CV32 7EP (GB); JONES, Matthew, Warwick Warwickshire CV34 6AU (GB); MARSH, William, Buckingham Buckinghamshire MK18 4HZ (GB); BUTLER, Joseph, Rugby Warwickshire CV21 4DU (GB)
(74) Representative: Keil & Schaafhausen Patentanwälte PartGmbB
(86) International application number: PCT/EP2014/056964
(87) International publication number: WO 2014/166886

(56) References cited:
- WO-A1-03/011374
- WO-A1-2006/089767
- WO-A1-2007/017052
- WO-A1-2007/017052
- WO-A1-2007/030957
- WO-A1-2012/084720
- US-A1- 2006 153 693
- US-A1- 2011 054 412

## Description

The present invention relates to a limiter mechanism which is suitable for an injection device, especially a pen type drug delivery device. The mechanism comprises a housing, a dose setting member, which during dose setting rotates relative to the housing in a first direction and which during dose dispensing rotates relative to the housing in a second opposite direction, and a limiting element for limiting the rotational movement of the dose setting member between a zero dose position and a maximum dose position. Further, the present invention relates to an injection device comprising such a limiter mechanism and a cartridge containing a medicament.

Pen type drug delivery devices have application where regular injection by persons without formal medical training occurs. This may be increasingly common among patients having diabetes where self-treatment enables such patients to conduct effective management of their disease. In practice, such a drug delivery device allows a user to individually select and dispense a number of user variable doses of a medicament. The present invention is not directed to so called fixed dose devices which only allow dispensing of a predefined dose without the possibility to increase or decrease the set dose.

There are basically two types of drug delivery devices: resettable devices (i.e., reusable) and non-resettable (i.e., disposable). For example, disposable pen delivery devices are supplied as self-contained devices. Such self-contained devices do not have removable pre-filled cartridges. Rather, the pre-filled cartridges may not be removed and replaced from these devices without destroying the device itself. Consequently, such disposable devices need not have a resettable dose setting mechanism. The present invention is in general applicable for both types of devices, i.e. for disposable devices as well as for reusable devices.

A further differentiation of drug delivery device types refers to the drive mechanism: There are devices which are manually driven, e.g. by a user applying a force to an injection button, devices which are driven by a spring or the like and devices which combine these two concepts, i.e. spring assisted devices which still require a user to exert an injection force. The spring-type devices involve springs which are preloaded and springs which are loaded by the user during dose selecting. Some stored-energy devices use a combination of spring preload and additional energy provided by the user, for example during dose setting. EP 2 198 903 A1 discloses a motor mechanism for a drug delivery device with a spring in the form of a strip of spring metal sheet attached to two drums.

These types of pen delivery devices (so named because they often resemble an enlarged fountain pen) are generally comprised of three primary elements: a cartridge section that includes a cartridge often contained within a housing or holder; a needle assembly connected to one end of the cartridge section; and a dosing section connected to the other end of the cartridge section. A cartridge (often referred to as an ampoule) typically includes a reservoir that is filled with a medication (e.g., insulin), a movable rubber type bung or stopper located at one end of the cartridge reservoir, and a top having a pierceable rubber seal located at the other, often necked-down, end. A crimped annular metal band is typically used to hold the rubber seal in place. While the cartridge housing may be typically made of plastic, cartridge reservoirs have historically been made of glass.

The needle assembly is typically a replaceable double-ended needle assembly. Before an injection, a replaceable double-ended needle assembly is attached to one end of the cartridge assembly, a dose is set, and then the set dose is administered. Such removable needle assemblies may be threaded onto, or pushed (i.e., snapped) onto the pierceable seal end of the cartridge assembly.

The dosing section or dose setting mechanism is typically the portion of the pen device that is used to set (select) a dose. During an injection, a spindle or piston rod contained within the dose setting mechanism presses against the bung or stopper of the cartridge. This force causes the medication contained within the cartridge to be injected through an attached needle assembly. After an injection, as generally recommended by most drug delivery device and/or needle assembly manufacturers and suppliers, the needle assembly is removed and discarded.

In the following, the distal end of an injection device or drive mechanism is referred to as the end where a cartridge and e.g. a needle are located, whereas the opposite end is the proximal end. A dose button may be provided at the proximal end.

The general function of a device as defined above is to set a dose and to subsequently dispense the set dose. Dose setting (dose dialing) usually requires a user to manipulate one element of the drive mechanism, preferably to rotate a dial member e.g. via a dial grip. During dose dispensing the dial member may move, e.g. rotate, back to its original position wherein a drive member, which is not actuated during dose setting, is moved together with the dial member during dose dispensing. The movement of the drive member may be a rotation, a displacement in the distal direction or a combined movement e.g. along a helical path. The drive member may act on a piston rod, e.g. a lead screw, for expelling medicament from a cartridge during dose dispensing.

In addition to this basic function of a drive mechanism it is in some cases preferred to allow a resetting of an already set dose, i.e. a correction or a deselecting of a dose. Preferably the user simply has to rotate the dial member, e.g. via a dial grip, in the opposite direction compared to the rotation during dose setting. Preferably, the drive member is not actuated during dose resetting, either.

At the beginning of dose setting, the mechanism is usually in a zero dose position, i.e. the previous dose has been fully administered and no new dose has been dialed. The user may set a dose up to a maximum dose which is defined by the mechanism, for example by providing an end stop which prevents setting of a higher dose. Typically, a maximum settable dose is 60, 80, 100 or 120 units of a medicament. During dose resetting, an already set dose may be reduced down to the zero dose position of the device. It is important to provide a defined zero dose position and a defined maximum dose position to increase accuracy of the device and to prevent overdosing.

To allow rotation of components of the drive mechanism, it is preferred if the components are mainly located concentrically about a common longitudinal axis of the drive mechanism. Thus, the components may have a tubular or sleeve-like shape. For example, the drive member and a dial or dose setting member may each be a tubular element. Some components may be provided surrounding other components fully or partly or may be provided one behind another.

WO 2007/017052 A1, US 2006/153693 A1, WO 03/011374 A1, WO 2007/030957 A1 and US 2011/054412 A1 disclose drug delivery devices with a display and/or dose setting member which is axially displaceable relative to the housing during use of the device. This results in a device requiring a relatively large space in the axial direction which may be undesired for some users.

An injection device is known from EP 1 974 761 B1 wherein during dose setting, dose resetting and dose dispensing a dose grip and a dose dial sleeve rotate with respect to a housing and a housing insert between a zero dose position and a maximum dose position. A drive sleeve is provided with a clutch which is arranged such that a relative rotation of the drive member about an axis of rotation with respect to the housing is allowed during dose setting and is prevented during dose dispensing. A limit of travel during dose setting is provided by a radial stop on the dose dial sleeve which engages a stop provided on the housing to prevent further movement. Once a dialed dose has been dispensed, the dose dial sleeve is prevented from further rotation by contact of a plurality of members extending from the dose dial grip with a corresponding plurality of stops formed in the housing.

In the injection device of EP 1 974 761 B1 the dose dial sleeve is in threaded engagement with the housing such that the dose dial sleeve extends from the housing during dose setting and is wound back into the housing during dose dispensing. Further, it is required that a user exerts an axial force during dose dispensing which might be a drawback for a manually impaired user.

It is an object of the present invention to provide an improved alternative to the above solutions.

This object is solved by a limiter mechanism with the features of claim 1. According to a first embodiment of the present invention, the mechanism comprises a housing, a dose setting member, which during dose setting rotates relative to the housing in a first direction and which during dose dispensing rotates relative to the housing in a second opposite direction, and a limiting element limiting the rotational movement of the dose setting member between a zero dose position and a maximum dose position. The limiting element is movable on a first path axially displaceable relative to one of the housing and is movable on a second, helical path relative to the dose setting member. At least one of one of the first path and the second path has an end stop limiting the relative movement of the limiting element. In other words, a relative rotation of the dose setting member and the housing causes the limiting element to travel on both paths with the movement along the helical path results in a displacement on the axial path or vice versa. Thus, if the movement in one of these paths is stopped by an end stop, further rotation of the dose setting member relative to the housing is prevented. This is used to define a zero dose limit and a maximum dose limit.

Preferably, the first path has a design such that the limiting element is rotationally constrained to one of the housing or the dose setting member. However, as an alternative, the first path could be a twisted spline, which is in practice a helical path with a very large pitch. This may not then be a rotationally constrained limiting element. The invention requires that the limiting element must move along two helical paths, with different pitches. In this respect, a purely axial spline is considered as a helical path with an infinite pitch.

By providing an additional element limiting movements in the device at the zero dose position and at the maximum dose position during dose setting and dose dispensing, different designs of the device may be chosen, which e.g. do not require that the dose setting member protrudes from the housing during dose setting. In addition, the force required during dose dispensing may be reduced. The present invention is based on the idea to de-couple a limiting element from a dose setting member such that a relative movement between the dose setting member and the limiting element is allowed. Such a relative movement may be a relative displacement and/or a relative rotation.

According to the present invention, the limiting element is a nut with an internal thread engaging an external thread on the dose setting member. In this embodiment it is preferred if the limiting element is provided with splines at its radially outer surface which are guided in corresponding splines provided on an inner surface of the housing.

As an alternative, according to an embodiment of another limiting mechanism, the limiting element is a nut with an external thread engaging an internal thread of the housing. In this embodiment it is preferred if the limiting element is provided with splines at its radially inner surface which are guided in corresponding splines provided on an outer surface of the dose setting member.

The two above embodiments are not limited to the design of the limiting element as a full sleeve- or ring-like nut. Moreover, a half nut may be provided extending about 180°. As an alternative to a splined engagement any other suitable means may be provided allowing guiding the limiting element in the housing in a rotationally constrained manner, e.g. a protrusion running in a notch or groove, corresponding teeth, or the like.

To limit movements of the limiter mechanism, a first counter stop is provided at a first end of the limiting element and a second counter stop is provided at an opposite, second end of the limiting element. Preferably the stops are located at the distal and proximal ends of the limiting element, respectively.

According to a further development of this idea, one of the housing or the dose setting member is provided with a first end stop and a second end stop which are located such that the limiter mechanism is in its zero dose position if the first end stop abuts the first counter stop and the limiter mechanism is in its maximum dose position if the second end stop abuts the second counter stop. As an alternative, one of the end stops may be provided on the housing and the other on the dose setting member. The invention is not limited to embodiments having both the first end stop and the second end stop on the same component.

The end stops may simply abut the counter stops in an axial direction during relative movement. However, it is preferred if the first end stop and the first counter stop and/or the second end stop and the second counter stop are rotational stops. Further, end stops and counter stops may be provided by the threaded engagement, i.e. movement is limited by the nut reaching the end of a helical track of a thread.

According to a preferred embodiment, the dose setting member is not axially displaced or wound out of the housing. In other words, the length of the injection device does not change during dose setting and dose dispensing. According to the present invention, the dose setting member is axially constrained within the housing, e.g. by a snap connection of an annular bead and a corresponding annular notch.

The limiter mechanism may further comprise a drive member engaging a piston rod, e.g. a threaded lead screw, such that a rotation of the drive member causes an axial movement of the piston rod. Thus, a threaded engagement may be provided between the piston rod and the housing. If the drive member is not axially movable, there may be a threaded engagement between the piston rod and the drive member. Preferably, the drive member rotates relative to the housing during dose dispensing and is rotationally constrained to the housing during dose setting.

Further, the limiter mechanism may comprise a clutch provided interposed between the dose setting member and the drive member, wherein the clutch allows relative rotational movement between the dose setting member and the drive member during dose setting and prevents relative rotational movement between the dose setting member and the drive member during dose dispensing. Thus, as the drive member engages the piston rod, the piston rod will not move during dose setting or dose resetting, but only during dose dispensing.

The drive member may be axially movable between a dose setting position, preferably a proximal position, in which the drive member is rotationally constrained to the housing, and a dose dispensing position, preferably a distal position, in which the drive member is rotationally de-coupled from the housing. Thus, irrespective of for example a spring load acting on the drive member, the drive member is prevented from rotation in its dose setting position.

Preferably, the clutch comprises a clicker mechanism. For example, the dose setting member may be axially constrained within the housing but is free to rotate, resisted by sprung detent features between the dose setting member and the drive member or a component connected to the drive member, e.g. a spool.
These detent features provide positive feedback to the user during dialing.

Further, a clicker mechanism with a clicker arm may be provided, which arm is rotationally constrained to the housing, wherein the drive member comprises teeth interacting with the clicker arm in the dose dispensing position of the drive member. This clicker mechanism is active during dose dispensing. These teeth may be used to rotationally constrain the drive member to the housing as mentioned above, e.g. by engagement of the teeth with corresponding teeth of the housing.

Preferably, the clicker arm is elastically displaceable in a radially outwards direction to allow the clicker arm to bump over the teeth of the rotating drive member during dose dispensing. If the stiffness of the clicker arm varies over its length in the longitudinal direction, the retarding force required to overcome engagement of the clicker arm and the teeth changes with the relative axial position of the clicker arm and the teeth of the drive member. In other words, this clicker mechanism may have the additional function of a speed control. For example, due to the higher stiffness of the clicker arm in a proximal part thereof, interaction of the teeth of the drive member with this stiffer part has a higher retarding effect compared with interaction of the teeth of the drive member with a more compliant distal part of the clicker arm. It is preferred if the axial position of the drive member may be amended by actuation of a trigger or dose button. Further, the stiffness may also vary in radial direction orthogonal to longitudinal direction, for example with an L shaped geometry

The drive mechanism may further comprise a reverse wound flat spiral spring as a power reservoir. Preferably, the spring has a first end attached to a first spool and a second end attached to a second spool with one of the spools being coupled to the drive member. In the "fully charged" state (no doses dispensed from cartridge) the majority of the spring is wound onto an output spool with a small length back-wound around a storage spool. As the spring is discharged during dose dispensing it winds onto the storage spool and off the output spool.

According to a preferred embodiment, the limiter mechanism comprises a flat spring arranged between the housing and the drive member, e.g. on spools as explained above, a further clutch rotationally coupling the drive member and the housing during dose setting and de-coupling the drive member to the housing during dose dispensing and a button provided on the proximal end of the limiter mechanism. Actuation of the button preferably causes axial displacement of the drive member, coupling of the clutch and de-coupling of the second clutch.

To provide a visual indication of a set dose, numerals or markings are provided on a component of the limiter mechanism which are visible from the outside of the housing. According to the present invention, at least the dose setting member is provided with such numerals or markings. In addition, a further element may be provided with such numerals or markings. In more detail, the dose display may be divided into "tens" and "units", each shown on a separate wheel and which index at different rates. The units may be printed directly onto the dose setting member, e.g. a dial sleeve, and, therefore, index as the dose setting member is rotated. A transfer gear may link the dose setting member and a tens wheel to increment the tens wheel once for every 10 units indexed on the dose setting member. If a clicker is provided in the clutch, the detent features may align the dose setting member with the housing e.g. via a spool and the drive member so the units of the dose display align with the dose window accurately.

To prevent an underdosage or a malfunction, the drug delivery device may comprise a last dose protection mechanism for preventing the setting of a dose, which exceeds the amount of liquid left in a cartridge. For example, the last dose protection mechanism comprises a nut member located interposed between the drive member and the dose setting member or any other component which rotates during dose setting and dose dispensing. In a preferred embodiment, the dose setting member rotates during dose setting and during dose dispensing, whereas the drive member only rotates during dose dispensing together with the dose setting member. Thus, in this embodiment, the nut member will only move during dose setting and will remain stationary with respect to these components during dose dispensing. Preferably, the nut member is threaded to the dose setting member and splined to the drive member. As an alternative, the nut member may be threaded to the drive member and may be splined to the dose setting member. The nut member may be a full nut or a part thereof, e.g. a half nut.

The object of the present invention is further solved by an injection device according to claim 15. An injection device may comprise a limiter mechanism as mentioned above and a cartridge containing a medicament. Preferably, the flat spring is pre-tensioned to store the energy required to dispense the whole contents of the cartridge.

The term "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, , an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyhepta¬decanoyl) human insulin.

Exendin-4 for example means Exendin-4(1-39), a peptide of the sequence H His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (-150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (CH) and the variable region (VH). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCI or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

Non-limiting, exemplary embodiments of the invention will now be described with reference to the accompanying drawings, in which:
Figure 1 shows an exploded view of an injection device comprising a limiter mechanism according to a first embodiment of the invention,
Figure 2 shows a section view of the limiter mechanism of Figure 1 during dose setting,
Figure 3 shows an enlarged detail of the limiter mechanism of Figure 1 in the zero dose position,
Figure 4 shows an enlarged detail of the limiter mechanism of Figure 1 in the maximum dose position,
Figure 5 shows an enlarged detail of the limiter mechanism of Figure 1,
Figure 6 shows an enlarged detail of the limiter mechanism of Figure 1,
Figure 7 shows an enlarged detail of the limiter mechanism of Figure 1,
Figure 8 shows an enlarged detail of the limiter mechanism of Figure 1,
Figure 9 shows a spring of the limiter mechanism of Figure 1,
Figure 10a shows the spring of Figure 9 in its fully charged state,
Figure 10b shows the spring of Figure 9 in its fully discharged state,
Figure 11 shows an enlarged detail of the limiter mechanism of Figure 1,
Figure 12 shows an enlarged detail of the limiter mechanism of Figure 1,
Figure 13 shows an exploded view of an injection device comprising a limiter mechanism according to a second embodiment of the invention,
Figure 14 shows a section view of the limiter mechanism of Figure 13 during dose setting,
Figure 15 shows an enlarged section view of the limiter mechanism of Figure 13 during dose dispensing
Figure 16 shows an enlarged detail of an injection device according to a third embodiment of the invention,
Figure 17a shows the detail of Figure 16 in the dose setting mode,
Figure 17b shows the detail of Figure 16 in the dose dispensing mode, and
Figure 17c shows the detail of Figure 16 in the dose dispensing mode.

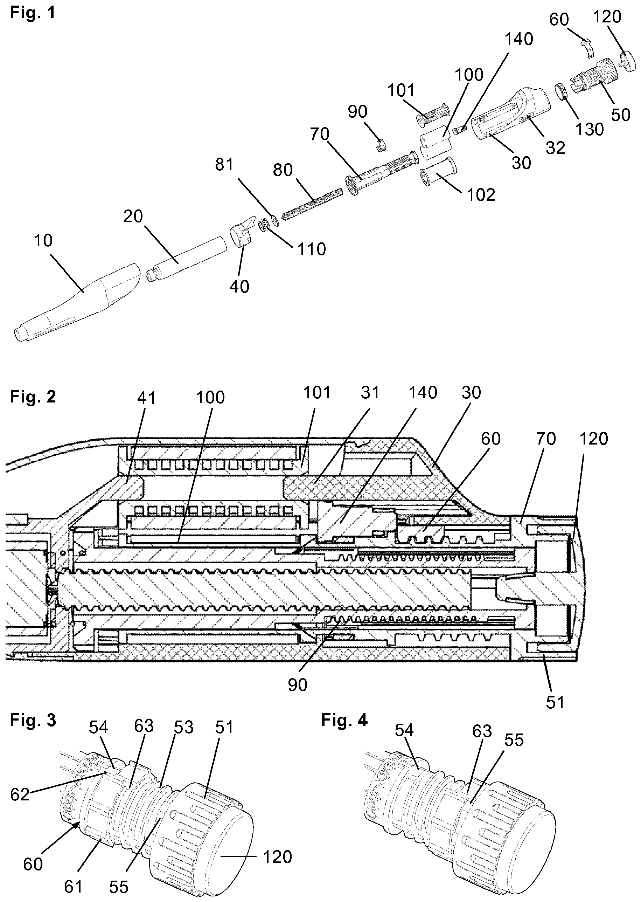

An injection device 1 according to the present invention is shown in Figure 1 in an exploded view. The injection device comprises a cartridge holder 10, a cartridge 20 and a limiter mechanism. The limiter mechanism comprises a housing with an outer housing 30 and an inner housing 40, a dial sleeve as a dose setting member 50 with a dial grip 51, a limiting element 60, a tubular drive member 70, a lead screw 80, a bearing 81, a nut 90, a drive spring 100 with a storage spool 101 and an output spool 102, a return spring 110, a dose button 120, a display wheel 130 and a gear wheel 140. With the exception of the gear wheel 140, the storage spool 101 and the spring 100, all components are located concentrically about a common principle axis of the mechanism. In more detail, the drive member 70 surrounds the lead screw 80, the output spool 102 and the dose setting member 50 surround the drive member 70, and the limiting element 60 and the display wheel 130 surround the dose setting member 50. Further, the nut 90 is located between the drive member 70 and the dose setting member 50.

Figures 1 and 2 show cross-sectional views of the limiter mechanism concept incorporated into a pen injector. A medicament cartridge 20 is housed within the cartridge holder 10. The cartridge holder 10 is rigidly constrained in a body defined by the proximal housing part 30 and the distal housing part 40. The cartridge holder 10 provides location and containment of the medicament cartridge 20 and partial protection of the spring 100 and the storage spool 101.

The distal end of the lead screw 80 connects to the bearing 81 to permit relative rotation but prevent axial separation. The distal face of the bearing 81 abuts a bung of the medicament cartridge 20. It is the lead screw 80 and bearing 81 assembly that drives the bung axially in order to deliver medicament. The lead screw 80 has a thread running along its length, which may be a twin-start thread, in addition to splines 82, which may be a plurality of splines

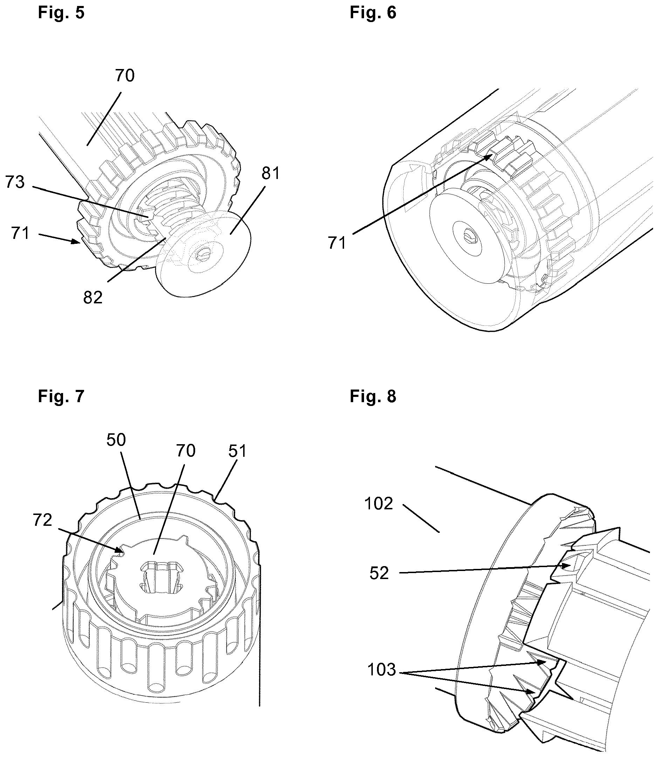

The distal end of the lead screw 80 is threaded to a thread insert which is formed by the inner housing 40 and the splines 82 in the lead screw 80 engage with axial ribs 73 of the drive member 70 as shown in Figure 5. Rotation of the drive member 70 thus causes the lead screw 80 to advance axially through the thread insert (moving the bung).

The inner housing 40 with the thread insert is rigidly constrained in the outer housing 30 and provides thread connection to lead screw 80, axial abutment to the trigger spring 110, an axial end stop for the drive member 70, support of the storage spool 101 distal end and location features for the cartridge holder 10.

The trigger spring 110 acts between the inner housing 40 and the drive member 70 and provides a reaction force on the drive member 70 to return it to an "at rest" state which is depicted in Figure 2. As an alternative to the embodiment with a separate trigger spring 110, a trigger spring function may be provided integrated for example in the housing 40 or the drive member 70.

The drive member 70 provides the mechanical connection between the spring 100 and lead screw 80 to transfer the torque that delivers the drug to the user. In the "at rest" state (0 units dialled, trigger button 120 released) the drive member 70 is splined to the outer housing 30 at the distal end by a ring of teeth 71. Corresponding splines on the outer housing 30 are depicted in Figure 6. These spline features 71 react the spring 100 torque and prevent uncontrolled release of the spring energy. In other words, a clutch is provided between the outer housing 30 and the drive member 70, which is closed (preventing relative rotation) during dose setting or dose resetting and which is open (allowing relative rotation) during dose dispensing.

The outer surface of the drive member 70 engages with splines on the inner surface of the output spool 102. The drive member 70 and the output spool 102 remain rotationally linked at all times.

In the "triggered" condition (trigger button 120 depressed), the drive member 70 is rotationally coupled to the dose setting member 50 through additional spline features 72 located at the proximal end of the drive member 70 as shown in Figure 7. Thus, a further clutch is provided between the drive member 70 and the dose setting member 50, which is open (allowing relative rotation) during dose setting or dose resetting and which is closed (preventing relative rotation) during dose dispensing.

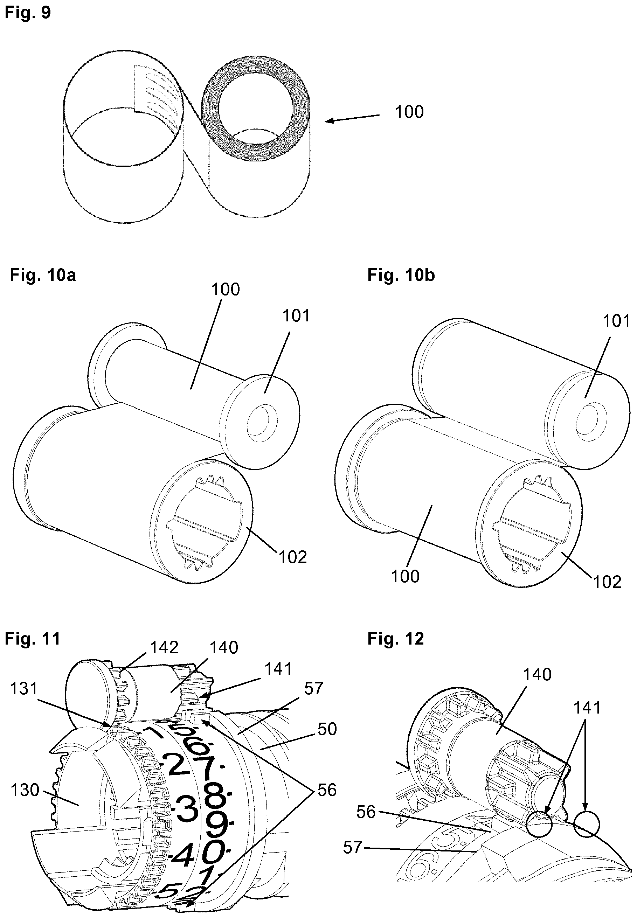

The spring 100 is a reverse wound flat spiral spring. Figure 9 shows an illustrative example of the spring in a partially charged state, with the spools omitted for clarity. In Figures 10a and 10b, the spring 100 is wound around two cylindrical spools 101, 102. In the "fully charged" state shown in Figure 10a (no doses dispensed from cartridge 20) the majority of the spring 100 is back-wound onto the output spool 102 with a small length naturally-wound around the storage spool 101. As the spring 100 is discharged (during dose dispense) it winds onto the storage spool 101 and off the output spool 102. The spring 100 remains connected to both the storage spool 101 and output spool 102 at all times. Features on the output spool 102 engage with corresponding features on the end of the spring 100.. The spring 100 is not mechanically anchored to the storage spool 101 since the natural curvature of the spring strip ensures tight coiling around the storage spool 101. Once there are a couple of wraps of strip material on the output spool, the force at the achorage is negligible. The torque is generated by a more complex combination of the strip un-bending from its back-wound state as it leaves the output spool and further un-bending of the strip as assumes its natural formed diameter as it winds itself onto the storage spool.

The storage spool 101 is positioned off axis to the lead screw 80 by a location boss 41 of the inner housing 40 at the distal end and a location boss 31 of the outer housing 30 at the proximal end. The location bosses 31, 41 permit free rotation of the storage spool 101 whilst constraining axial translation.

The trigger button 120 is axially, but not rotationally, constrained within the proximal end of the drive member 70. User input force applied in the distal direction to the trigger button 120 is reacted through the drive member 70 by the trigger spring 110. The end stop for this user input is provided by the inner housing 40 acting on the distal end of the drive member 70. On release of the trigger button 120 the trigger spring 110 returns the drive member 70 in the proximal direction to the "at rest" position (Figure 2).

The dose setting member 50 is positioned towards the proximal end of the device allowing setting and cancelling (resetting) of a dose by rotation of the grip features 51 on it that can be accessed by the user. The dose setting member 50 is axially constrained within the outer housing 30 but is free to rotate, resisted by sprung detent features 52, 103 between the dose setting member 50 and output spool 102 (see Figure 8). These detent features 52, 103 provide positive feedback to the user during dialling and align the dose setting member 50 with the outer housing 30 via the output spool 102 and drive member 70 so the units of the dose display which is provided on the dose setting member 50 align with a dose window of the outer housing 30 accurately.

The limiting element 60 is a dose nut with an internal thread which engages a threaded section 53 on the dose setting member 50 and external splines 61 meshing splines of the outer housing 30. The limiting element 60 is thus rotationally constrained to the outer housing 30 but axially displaceable relative to the outer housing 30. In the embodiment of Figures 1 to 12, the limiting element 60 has the form of a half nut. However, the limiting element 60 may also be provided as a full nut. Rotation of the drive member 70 by the spring 100 is limited by the limiting element 60 via the dose setting member 50. The limiting element 60 provides the zero unit stop in its most distal position (Figure 3) and maximum dose unit stop when in its most proximal position (Figure 4). However, as an alternative, the limiting element 60 may run in the opposite direction. Abutments 54, 55 on the dose setting member 50 engage corresponding abutments 62, 63 on the limiting element 60 to create positive stop positions.

Because the outer surface of the limiting element 60 is splined to the housing 30 and the inner surface of the limiting element 60 is threaded to the dose setting member 50, a clockwise (CW) rotation of the dose setting member 50 translates the limiting element 60 in the proximal direction. Since the limiting element 60 provides the end of dose stop, the features 54, 62 and 55, 63 interacting at this point are designed to be particularly robust to minimize the risk of failure (see Figures 3 and 4).

The dose display mechanism shows the dose set that has been set by the user and also the dose remaining as a dose is dispensed. Three components interact to provide numerical display through a transparent window 32 component secured within the outer housing 30. The dose display is divided into "tens" and "units", each shown on a separate wheel and which index at different rates. The units are printed directly onto the dose setting member 50 and, therefore, index as the dose setting member 50 is rotated. The gear wheel 140 acts as a transfer gear which links the dose setting member 50, i.e. the units, and the display wheel 130 which is the tens wheel to increment the display wheel 130 once for every 10 units indexed on the dose setting member 50.

The dose setting member 50 incorporates two pairs of gear teeth 56 at diametrically opposing positions (see Figure 11). These teeth 56 engage the smaller pitch circle diameter (PCD) gear teeth 141 of the gear wheel 140 and create intermittent rotation of the gear wheel 140 twice per dose setting member 50 revolution. The display wheel 130 incorporates gear teeth 131 around the entire circumference which are always meshed with the larger PCD gear teeth 142 of the gear wheel 140.

The dose setting member 50 has 20 numbers printed around its circumference (0,1,2...8,9,0,1,2...8,9). The positions of the gear teeth 56 correspond to the index of the display wheel 130 (tens) in relation to the rotation of the dose setting member 50. As the units display indexes from "9" to "0" the teeth 56 on the dose setting member 50 engage with the gear wheel 140 and create a one quarter turn of the transfer gear wheel 140. This rotation delivers a one-twelfth rotation of the tens display wheel 130 corresponding to the pitch of the printed numbers.

Free rotation of the transfer gear wheel 140 is prevented by removing half the length of the gear teeth 141 at alternate positions at the extreme proximal end of the gear wheel 140 (see Figure 12). This modified gear profile interferes with a circumferential rib 57 on the dose setting member 50, fixing rotation of the gear wheel 140 when not in positive engagement with the dose setting member 50 gear teeth. Two reliefs in this rib 57 permit rotation of the gear wheel 140 corresponding to engagement of the dose setting member 50 gear teeth.

In this embodiment the maximum dose display possible is 129 although the mechanism may be limited to a lower maximum number of units, e.g. 80 units, by the limiting element 60. Altered gear ratios and frequency of tens display wheel 130 index provide options for alternative dose displays. In order to improve robustness of the gear engagement an alternative embodiment of the display mechanism modifies the modulus of the gear teeth. Replacing the pair of teeth 56 on the dose setting member 50 with a single tooth permits the use of larger gear teeth with greater height of engagement. The single relief in the dose setting member 50 circumferential rib (that prevents rotation of the tens display wheel 130 when not indexing) is replaced with a pair of reliefs either side of the single tooth.

In the following, operation of the limiter mechanism is explained in more detail: For dose setting, the dose setting member 50 is rotated by the user in a CW direction to set a dose. The dose can be cancelled by rotating the dose setting member 50 in a counter clockwise (CCW) direction either before any dispense or, alternatively, if the trigger button 120 is released mid-dispense, the remaining dose may be cancelled. The selected dose is displayed through the window 32 in the housing 30 via the dose display mechanism described previously. Irrespective of whether the dose setting member 50 is rotated CW or CCW the dose displayed will always indicate the dose to be dispensed. In addition, the dose display also decrements as the dose is dispensed and thus displays the dose remaining to be dispensed.

As the dose is dialled up the limiting element 60 is driven in the proximal direction along the threaded connection with the dose setting member 50. The dose setting member 50 can be rotated by the user in both CW and CCW directions when the limiting element 60 is not in contact with the zero dose or maximum dose stop abutments 54, 55 of the dose setting member 50. The end of dose abutment 54, 62 prevents CCW rotation of the dose setting member 50 below the 0 unit position. The maximum dose abutment 55, 63 prevents setting of a dose greater than the mechanism maximum, e.g. 80 units.

The detent feature 52, 103 between dose setting member 50 and output spool 102 controls the position of the dose setting member 50 to ensure that discrete units are selected and that the spline features between drive member 70 and dose setting member 50 are correctly aligned to permit spline meshing when the device is triggered.

During dose setting the drive member 70 is coupled to the outer housing 30 via splines (teeth 71) at its distal end and biased into engagement with these splines by the trigger spring 110. The drive member 70 is, therefore, fixed rotationally during dose set which, in turn prevents rotation of the output spool 102 and lead screw 80.

The mechanism incorporates a last dose nut 90 to prevent setting a dose greater than that which remains within the medicament cartridge. This is positioned between the dose setting member 50 and drive member 70 since the dose setting member 50 rotates relative to the drive member 70 during dose set and not during dispense. The nut 90 is splined to the inner surface of the dose setting member 50 and threaded to the drive member 70 such that CW rotation of the dose setting member 50 rotates the last dose nut 90 and translates it in the distal direction. As an alternative, the last dose nut 90 may be splined to the drive member 70 and threaded to the dose setting member 50. The last dose nut 90 is successively translated distally as doses are set and dispensed until the cartridge dose limit is reached. At this point the nut 90 contacts an abutment on the drive member 70 which prevents further CW rotation of the last dose nut 90 and, therefore, CW rotation of the dose setting member 50. The number of permissible rotations of the last dose nut 90 is determined by the capacity of the cartridge 20.

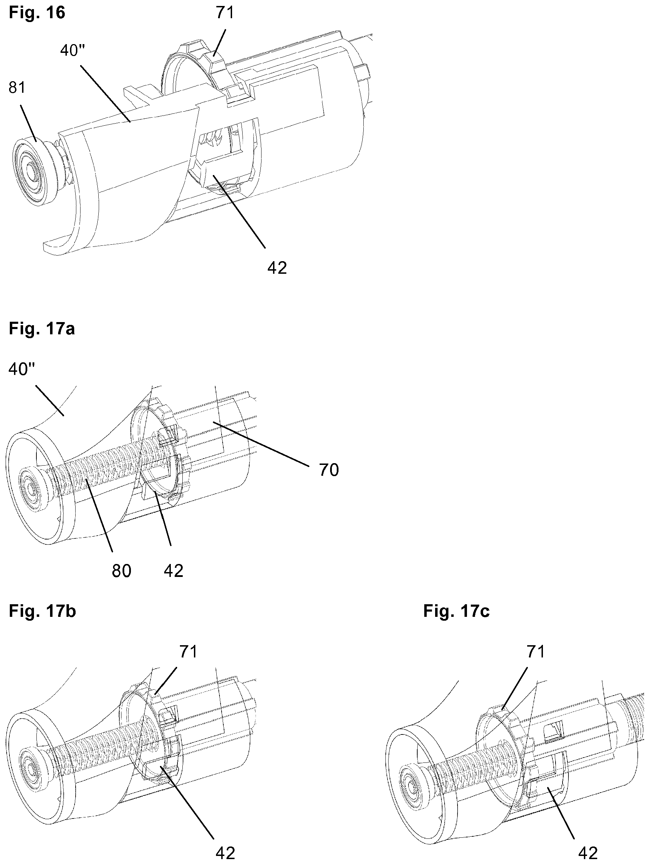

The device may be triggered by the user through application of an axial force on the trigger button 120 in the distal direction. The trigger button 120 acts on the drive member 70, translating the drive member 70 and last dose nut 90 in the distal direction, compressing the trigger spring 110. As the drive member 70 translates it first engages with the dose setting member 50 through splines 72 towards the proximal end of the device. At this stage (trigger button 120 mid position) the dose setting member 50 can no longer be rotated in either direction since the splines 71 at the distal end of the drive member 70 remain in engagement with the outer housing 30. This distal translation of the drive member 70 also engages a dispense feedback clicker feature (not shown in Figures 1 to 12, but in Figures 16 to 17c) of the outer housing 30 with the distal drive member 70 splines 71. Further distal translation of the trigger button 120 decouples the distal drive member 70 splines 71 from the outer housing 30, releasing rotation of the drive member 70 and spring 100 assembly.

On triggering, the torque generated by the spring 100 turns the drive member 70 and lead screw 80 via the output spool 102. Since the drive member 70 and dose setting member 50 are rotationally connected the dose setting member 50 also rotates during dispense in a CCW direction, translating the limiting element 60 distally. At the zero unit position the limiting element 60 contacts the abutment 54 on the thread to the dose setting member 50, preventing further rotation of the dose setting member 50, drive member 70, lead screw 80 and output spool, ending the dose dispense (Figure 3).

The trigger button 120 is subsequently released, re-engaging the spline features 71 between drive member 70 and housing 30 thus locking rotation of the drive member 70, lead screw 80 and output spool 102 independent of the limiting element 60 to dose setting member 50 stop feature. This allows the next dose to be set without immediate release of the spring 100. Aside from the lead screw 80 assembly, spring 100 assembly and nut 90 all other components in the device return to their original positions once the entire dose has completed dispense. In reality, the orientation of the drive member, dial grip and trigger button may be different than their original positions, but this may be overlooked as the components have rotational symmetry.

The spline teeth in the housing 30 that engage with the drive member 70 are angled so the drive member 70 is turned against the spring torque as they re-engage when the trigger button 120 is released. Back-winding the drive member 70 retracts the lead screw 80 assembly and ensures that the drive member 70 to housing 30 splines act as the end of dose stop in place of the limiting element 60. The back-winding of the drive member 70 removes the effect of clearances within the mechanism (as a result of designing for manufacturing tolerances or assembly) which could otherwise lead to slight advancement of the lead screw 80 and medicament dispense when the device is dialled for the subsequent dose.

The limiter mechanism provides a platform for the development of a range of pen injectors that provide delivery of a user variable medicament dose with relatively very low user setting torque and user injection force. There is potential for the variable dose to have any pre-defined maximum dose.

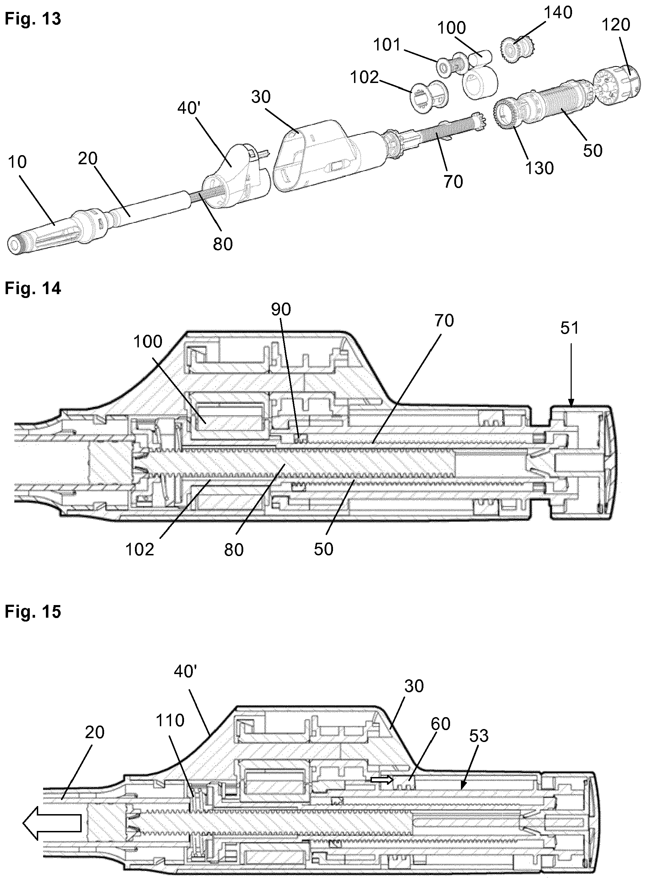

A second embodiment of a limiter mechanism is shown in Figures 13 to 15. The general construction and functions are very similar to those of the first embodiment depicted in Figures 1 to 12.

Main differences between the two embodiments are that the cartridge holder 10 is shorter and thus does not house the spring 100 and spool 101. Further, the housing comprises a proximal housing part 30 and a distal housing part 40' wherein the distal housing part 40' mainly fulfills the functions of the inner housing of the first embodiment, i.e. it forms a threaded insert for the lead screw 80, it provides a bearing for the trigger spring 110 and it is provided with splines forming a clutch with corresponding splines of the drive member 70. In addition, the transfer gear wheel 140 is made larger and is located coaxially with the storage spool 101. Further, the trigger button 120 is formed as an insert of a dose dial grip 51 which is rotationally coupled to the dose setting member 50 during dose setting and dose resetting.

Regarding the function, the limiter mechanism of the second embodiment allows setting of a larger maximum dose, e.g. 120 units compared to 80 units in the first embodiment. This is achieved by providing a longer threaded section 53 on the dose setting member 50 such that the track for the limiting element 60 is prolonged compared to the first embodiment.

Operation of the limiter mechanism of the second embodiment is as follows: During dose setting, the dose setting member 50 is rotated clockwise by the user via the dial grip 51. This causes the tubular dose setting member 50 to rotate, moving the limiting element 60 away from its zero units stop feature and increasing the dose displayed. The dose counter again consists of the dose setting member 50 sleeve with printed units and a tens display wheel 130 which is incremented by the action of the transfer gear wheel 140 once per revolution.

The last dose nut 90 rotates on a thread on the drive member 70 towards the last dose stop. The drive member 70 is locked by its splined engagement with the distal housing part 40' preventing it from being rotated by the spring 100.

To dispense a dose, the trigger button 120, i.e. the dose grip 51 is depressed. This disengages the grip 51 from the dose setting member 50 sleeve so that it does not rotate during dispense. Further, the drive member 70 is moved axially with the grip 51, engaging the splined interface between the drive member 70 and the dose setting member 50 and disengaging the splined engagement with the distal housing part 40' allowing the spring 100 to rotate the drive member 70. The drive member 70 winds the lead screw 80 forwards through the thread in the distal housing part 40' to advance the cartridge bung. The display mechanism and the limiting element 60 then return towards their zero dose positions.

At the end of dose dispensing, the limiting element 60 translates to the end of its travel and engages the zero unit dose stop with the dose setting member 50. Once the zero unit dose stop is engaged, it reacts the torque from the spring 100, preventing further rotation of the drive member 70. When the user releases the dose button 120, the trigger spring 110 force returns the drive member 70 into engagement with the distal housing part 40', and disengages the clutch between the drive member 70 and the dose setting member 50.

As an additional feature, an end of dose click is provided in the second embodiment. A spring arm 64 on the limiting element 60 rides over a zero unit stop feature and is deflected radially outwards as the dose setting member 50 rotates anti-clockwise towards the zero unit position anti position. As the zero unit stop passes the end of the spring arm 64, the stored energy is released moving the spring arm 64 inwards radially causing a click as it hits the dose setting member 50. When dialing up from 0 to 1 unit, the spring arm 64 is forced axially by a ramp on the dose setting member 50 and back into its unstressed position. This should not create any audible feedback.

As an alternative, a spring arm (not shown) on dose setting member 50 is deflected axially by a ramp feature on the limiting element 60 as the dose setting member 50 rotates anti-clockwise towards zero units. As the ramp feature passes the end of the spring arm, the stored energy is released, moving the spring arm axially causing a click as it hits the limiting element 60 thread form. When dialing up from 0 to 1 unit, the spring arm is forced axially by the opposite side of the ramp feature on the limiting element 60 falls off the edge, but does not contact a surface to cause a click. Throughout the rest of the stroke of the limiting element 60, the limiting element 60 traverses away from the spring arm and so they do not come into contact again.

A dispense clicker, which may also serve as a speed control mechanism, is described with respect to Figures 16 to 17c. This dispense clicker may be incorporated in any of the devices as described above, in which the drive member 70 is moved axially during dispense. This releases its splined engagement 71 with the housing part 40" (which may be similar to the distal or inner housing 40, 40'), allowing it to be rotated by the stored energy source (spring 100). The rotation of the drive member 70 causes the lead screw 80 to advance through a thread and dispense medicament.

The mechanism described here contains a radially-acting dispense clicker arm 42 formed within the housing part 40" (which may be a thread insert) that acts on teeth 71 on the drive member 70, which changes in stiffness as the drive member 70 moves in an axial direction. The stiffness of this clicker arm 42 determines the frictional drag on the drive member 70 and hence the speed at which the device dispenses the medicament, as the torque which drives drive member 70 is approximately constant regardless of the dialled dose or the remaining medicament in the cartridge 20.

The stiffness is highest (as embodied) when the dose button 120 is displaced by the smallest displacement necessary to release the spline features 71 allowing the drive member 70 to rotate and dispense the medicament. As the dose button 120 (and hence the drive member 70) is displaced further towards the mechanism body, the clicker stiffness is reduced so that the frictional drag torque decreases, allowing the drive member 70 to rotate at a higher speed.

The spline features (teeth 71) on the drive member 70 have two functions. They primarily lock the drive member 70 to the housing, when the pen is in dose select (dialing) mode (see Figure 17a). They also act as ratchet features over which the clicker arm 42 passes and detents into discrete single unit positions (Figures 17b and 17c). The profile of these teeth 71 in conjunction with the tip profile and cantilever characteristics of the ratchet arm 42 determines the drag torque applied by the dispense clicker.

The cantilevered clicker arm 42 is designed to work either primarily in bending or primarily in torsion, depending on the axial position of the drive member 70. Then the dose button 120 is pressed just enough to leave the dialling mode and enter dispense mode (drive member 70 clutch teeth 71 to thread insert are disengaged, see Figure 17b), the clicker arm 42 is in a pure bending regime. As the dose button 120 is pressed progressively further (see Figure 17c), the clicker arm 42 becomes more torsionally loaded. The L-shaped geometry of the cantilever means that the effective cantilever stiffness is significantly less in torsion compared with pure bending, therefore the clicker drag torque becomes less. The less-stiff clicker arm torque requires less energy for the clutch teeth 71 to overcome, so the resultant speed of dispense becomes greater as less energy is required to overcome the clicker mechanism and more of the stored energy is converted to driving the lead screw 80 forward, dispensing medicament.

The range of clicker torques available to limit dispense speed is continuous, but limited by the stroke of the drive member 70 which may be determined by clutch teeth 71 engagement required for robustness and for ergonomic considerations. The degree to which the clicker arm 42 retards speed of dispense can be tuned so that an acceptable range of speeds can be produced. The simple change in cantilever regimes allows a large range of clicker stiffnesses to be designed with a relatively short axial travel of the drive member 70, and so this feature can be applied to a range of mechanisms where a stored energy source is used.

An alternative embodiment (not shown) would have an increasing clicker torque as the dose button 120 is pressed by an increasing amount towards the mechanism body. This would produce slower dispense speed as the dose button 120 is pressed displaced further towards the mechanism body.

The stops mentioned above limiting movements of component parts during dose setting and/or dose dispensing are preferably rotational hard stops or radial stops. As an alternative axial stops may be provided.

## Claims

1. Limiter mechanism for an injection device, the mechanism comprising a housing (10, 30, 40; 40'), a dose setting member (50), which during dose setting rotates relative to the housing (10, 30, 40; 40') in a first direction and which during dose dispensing rotates relative to the housing (10, 30, 40; 40') in a second opposite direction, and a limiting element (60) limiting the rotational movement of the dose setting member between a zero dose position and a maximum dose position,
**characterized in that** the limiting element (60) is a nut with an internal thread engaging an external thread on the dose setting member (50) which is guided in the housing (10, 30, 40; 40') is movable on a first path axially displaceable relative to the housing (10, 30, 40; 40') and is movable on a second, helical path (53) relative to the dose setting member (50), wherein at least one of one of the first path and the second path (53) has an end stop (54, 55) limiting the relative movement of the limiting element (60), and wherein the dose setting member (50) is provided with markings visible from the outside of the housing (10, 30, 40; 40') adapted for providing a visual indication of a set dose, and is axially constrained within the housing (10, 30, 40; 40').

2. Limiter mechanism according to claim 1, wherein the limiting element (60) is provided with splines (61) at its radially outer surface which are guided in corresponding splines provided on an inner surface of the housing (10, 30, 40; 40').

3. Limiter mechanism according to any of the preceding claims, wherein a first counter stop (62) is provided at a first end of the limiting element (60) and a second counter stop (63) is provided at an opposite, second end of the limiting element (60).

4. Limiter mechanism according to claim 3, wherein one of the housing (10, 30, 40; 40') or the dose setting member (50) is provided with a first end stop (54) and a second end stop (55) which are located such that the limiter mechanism is in its zero dose position if the first end stop (54) abuts the first counter stop (62) and the limiter mechanism is in its maximum dose position if the second end stop (63) abuts the second counter stop (55).

5. Limiter mechanism according to any of the preceding claims, wherein the first end stop (54) and/or the second end stop (55) is a rotational stop.

6. Limiter mechanism according to any of the preceding claims, further comprising a drive member (70) engaging a piston rod (80) such that a rotation of the drive member (70) causes an axial movement of the piston rod (80).

7. Limiter mechanism according to claim 6, further comprising a clutch (72) provided interposed between the dose setting member (50) and the drive member (70), wherein the clutch (72) allows relative rotational movement between the dose setting member (50) and the drive member (70) during dose setting and prevents relative rotational movement between the dose setting member (50) and the drive member (70) during dose dispensing.

8. Limiter mechanism according to any of the claims 6 or 7, wherein the drive member (70) is axially movable between a dose setting position, in which the drive member (70) is rotationally constrained to the housing (10, 30, 40; 40'), and a dose dispensing position, in which the drive member (70) is rotationally de-coupled from the housing (10, 30, 40; 40').

9. Limiter mechanism according to any of the claims 6 to 8, further comprising a clicker mechanism with a clicker arm (42) which is rotationally constrained to the housing (10, 30, 40; 40'), wherein the drive member (70) comprises teeth (71) interacting with the clicker arm (42) in the dose dispensing position of the drive member (70).

10. Limiter mechanism according to claim 9, wherein the clicker arm (42) is elastically displaceable in a radially outwards direction, with the stiffness of the clicker arm (42) varying over its length in the longitudinal direction.

11. Limiter mechanism according to any of claims 6 to 10, further comprising a reverse wound flat spiral spring (100) as a power reservoir having a first end attached to a first spool (101) and a second end attached to a second spool (102), with one of the spools (102) being coupled to the drive member (70).

12. Limiter mechanism according to any of the preceding claims, wherein a further element (130) is provided with markings visible from the outside of the housing (10, 30, 40; 40') adapted for visual indication of the set dose.

13. Injection device comprising a limiter mechanism according to any of the preceding claims and a cartridge (20) containing a medicament.

## Patentansprüche

1. Begrenzermechanismus für eine Injektionsvorrichtung, wobei der Mechanismus ein Gehäuse (10, 30, 40; 40'), ein Dosiseinstellglied (50), das sich während der Dosiseinstellung relativ zu dem Gehäuse (10, 30, 40; 40') in eine erste Richtung dreht und sich während der Dosisabgabe relativ zu dem Gehäuse (10, 30, 40; 40') in eine zweite, entgegengesetzte Richtung dreht, und ein Begrenzungselement (60) aufweist, das die Drehbewegung des Dosiseinstellglieds zwischen einer Nulldosisposition und einer Maximaldosisposition begrenzt,
**dadurch gekennzeichnet, dass** das Begrenzungselement (60) eine Mutter mit einem ein Außengewinde an dem Dosiseinstellglied (50) in Eingriff nehmenden Innengewinde ist, die in dem Gehäuse (10, 30, 40; 40') geführt ist, auf einer ersten Bahn axial verschiebbar relativ zu dem Gehäuse (10, 30, 40; 40') beweglich ist und auf einer zweiten, schraubenförmigen Bahn (53) relativ zu dem Dosiseinstellglied (50) beweglich ist, wobei die erste Bahn und/oder die zweite Bahn (53) einen Endanschlag (54, 55) hat, der die Relativbewegung des Begrenzungselements (60) begrenzt, und wobei das Dosiseinstellglied (50) mit Markierungen versehen ist, die von außerhalb des Gehäuses (10, 30, 40; 40') sichtbar sind und dazu ausgeführt sind, eine visuelle Angabe einer eingestellten Dosis bereitzustellen, und axial in dem Gehäuse (10, 30, 40; 40') festgelegt ist.

2. Begrenzermechanismus nach Anspruch 1, wobei das Begrenzungselement (60) an seiner radial äußeren Fläche mit Keilzähnen (61) versehen ist, die in entsprechenden, an einer Innenfläche des Gehäuses (10, 30, 40; 40') vorgesehenen Keilzähnen geführt sind.

3. Begrenzermechanismus nach einem der vorhergehenden Ansprüche, wobei ein erster Gegenanschlag (62) an einem ersten Ende des Begrenzungselements (60) vorgesehen ist und ein zweiter Gegenanschlag (63) an einem gegenüberliegenden zweiten Ende des Begrenzungselements (60) vorgesehen ist.

4. Begrenzermechanismus nach Anspruch 3, wobei das Gehäuse (10, 30, 40; 40') oder das Dosiseinstellglied (50) mit einem ersten Endanschlag (54) und einem zweiten Endanschlag (55) versehen ist, die so angeordnet sind, dass der Begrenzermechanismus in seiner Nulldosisposition ist, wenn der erste Endanschlag (54) an dem ersten Gegenanschlag (62) anliegt, und der Begrenzermechanismus in seiner Maximaldosisposition ist, wenn der zweite Endanschlag (63) an dem zweiten Gegenanschlag (55) anliegt.

5. Begrenzermechanismus nach einem der vorhergehenden Ansprüche, wobei der erste Endanschlag (54) und/oder der zweite Endanschlag (55) ein Drehanschlag ist.

6. Begrenzermechanismus nach einem der vorhergehenden Ansprüche, ferner aufweisend ein Antriebsglied (70), das eine Kolbenstange (80) so in Eingriff nimmt, dass eine Drehung des Antriebsglieds (70) eine axiale Bewegung der Kolbenstange (80) veranlasst.

7. Begrenzermechanismus nach Anspruch 6, ferner aufweisend eine Kupplung (72), die zwischenliegend zwischen dem Dosiseinstellglied (50) und dem Antriebsglied (70) vorgesehen ist, wobei die Kupplung (72) eine relative Drehbewegung zwischen dem Dosiseinstellglied (50) und dem Antriebsglied (70) während der Dosiseinstellung gestattet und eine relative Drehbewegung zwischen dem Dosiseinstellglied (50) und dem Antriebsglied (70) während der Dosisabgabe verhindert.

8. Begrenzermechanismus nach einem der Ansprüche 6 oder 7, wobei das Antriebsglied (70) axial zwischen einer Dosiseinstellposition, in der das Antriebsglied (70) gegen Drehung gesichert an dem Gehäuse (10, 30, 40; 40') festgehalten ist, und einer Dosisabgabeposition, in der das Antriebsglied (70) von dem Gehäuse (10, 30, 40; 40') drehentkoppelt ist, beweglich ist.

9. Begrenzermechanismus nach einem der Ansprüche 6 bis 8, ferner aufweisend einen Klickermechanismus mit einem Klickerarm (42), der gegen Drehung gesichert an dem Gehäuse (10, 30, 40; 40') festgehalten ist, wobei das Antriebsglied (70) Zähne (71) aufweist, die in der Dosisabgabeposition des Antriebsglieds (70) mit dem Klickerarm (42) zusammenwirken.

10. Begrenzermechanismus nach Anspruch 9, wobei der Klickerarm (42) in eine radial nach außen gehende Richtung elastisch verschiebbar ist, wobei die Steifheit des Klickerarms (42) über seine Länge in der Längsrichtung variiert.

11. Begrenzermechanismus nach einem der Ansprüche 6 bis 10, ferner aufweisend eine umgekehrt aufgewickelte Flachspiralfeder (100) als ein Kraftspeicher mit einem ersten Ende, das an einer ersten Spule (101) angebracht ist, und einem zweiten Ende, das an einer zweiten Spule (102) angebracht ist, wobei eine der Spulen (102) an das Antriebsglied (70) gekoppelt ist.

12. Begrenzermechanismus nach einem der vorhergehenden Ansprüche, wobei ein weiteres Element (130) mit Markierungen versehen ist, die von außerhalb des Gehäuses (10, 30, 40; 40') sichtbar sind und zur visuellen Angabe der eingestellten Dosis ausgeführt sind.

13. Injektionsvorrichtung, aufweisend einen Begrenzermechanismus nach einem der vorhergehenden Ansprüche und eine Kartusche (20), die ein Medikament enthält.

## Revendications

1. Mécanisme limiteur pour un dispositif d'injection, le mécanisme comprenant un boîtier (10, 30, 40 ; 40'), un organe de réglage de dose (50), qui, au cours du réglage de dose, tourne par rapport au boîtier (10, 30, 40 ; 40') dans une première direction et qui, au cours de la distribution de dose, tourne par rapport au boîtier (10, 30, 40 ; 40') dans une deuxième direction opposée, et un élément de limitation (60) limitant le mouvement de rotation de l'organe de réglage de dose entre une position de dose nulle et une position de dose maximale,
**caractérisé en ce que** l'élément de limitation (60) est un écrou avec un filetage intérieur venant en prise avec un filetage extérieur sur l'organe de réglage de dose (50), qui est guidé dans le boîtier (10, 30, 40 ; 40'), peut être déplacé sur une première trajectoire déplaçable axialement par rapport au boîtier (10, 30, 40 ; 40'), et peut être déplacé sur une deuxième trajectoire hélicoïdale (53) par rapport à l'organe de réglage de dose (50), au moins l'une parmi la première trajectoire et la deuxième trajectoire (53) ayant une butée de fin de course (54, 55) limitant le mouvement relatif de l'élément de limitation (60), et l'organe de réglage de dose (50) étant pourvu de marquages visibles depuis l'extérieur du boîtier (10, 30, 40 ; 40'), prévus pour fournir une indication visuelle d'une dose réglée, et étant retenu axialement à l'intérieur du boîtier (10, 30, 40 ; 40').

2. Mécanisme limiteur selon la revendication 1, dans lequel l'élément de limitation (60) est pourvu de cannelures (61) au niveau de sa surface radialement extérieure, lesquelles sont guidées dans des cannelures correspondantes prévues sur une surface intérieure du boîtier (10, 30, 40 ; 40').

3. Mécanisme limiteur selon l'une quelconque des revendications précédentes, dans lequel une première contre-butée (62) est prévue au niveau d'une première extrémité de l'élément de limitation (60) et une deuxième contre-butée (63) est prévue au niveau d'une deuxième extrémité opposée de l'élément de limitation (60).

4. Mécanisme limiteur selon la revendication 3, dans lequel l'un parmi le boîtier (10, 30, 40 ; 40') et l'organe de réglage de dose (50) est pourvu d'une première butée de fin de course (54) et d'une deuxième butée de fin de course (55) qui sont situées de telle sorte que le mécanisme limiteur soit dans sa position de dose nulle si la première butée de fin de course (54) bute contre la première contre-butée (62) et que le mécanisme limiteur soit dans sa position de dose maximale si la deuxième butée de fin de course (63) bute contre la deuxième contre-butée (55).

5. Mécanisme limiteur selon l'une quelconque des revendications précédentes, dans lequel la première butée de fin de course (54) et/ou la deuxième butée de fin de course (55) est/sont une butée de rotation.

6. Mécanisme limiteur selon l'une quelconque des revendications précédentes, comprenant en outre un organe d'entraînement (70) venant en prise avec une tige de piston (80) de telle sorte qu'une rotation de l'organe d'entraînement (70) provoque un mouvement axial de la tige de piston (80).

7. Mécanisme limiteur selon la revendication 6, comprenant en outre un embrayage (72) interposé entre l'organe de réglage de dose (50) et l'organe d'entraînement (70), l'embrayage (72) permettant un mouvement de rotation relatif entre l'organe de réglage de dose (50) et l'organe d'entraînement (70) au cours du réglage de dose et empêchant un mouvement de rotation relatif entre l'organe de réglage de dose (50) et l'organe d'entraînement (70) au cours de la distribution de dose.

8. Mécanisme limiteur selon l'une quelconque des revendications 6 et 7, dans lequel l'organe d'entraînement (70) peut être déplacé axialement entre une position de réglage de dose dans laquelle l'organe d'entraînement (70) est solidaire en rotation du boîtier (10, 30, 40 ; 40'), et une position de distribution de dose dans laquelle l'organe d'entraînement (70) est désaccouplé en rotation du boîtier (10, 30, 40 ; 40').

9. Mécanisme limiteur selon l'une quelconque des revendications 6 à 8, comprenant en outre un mécanisme de cliquet avec un bras de cliquet (42) qui est solidaire en rotation du boîtier (10, 30, 40 ; 40'), l'organe d'entraînement (70) comprenant des dents (71) interagissant avec le bras de cliquet (42) dans la position de distribution de dose de l'organe d'entraînement (70).

10. Mécanisme limiteur selon la revendication 9, dans lequel le bras de cliquet (42) peut être déplacé élastiquement dans une direction radialement vers l'extérieur, la rigidité du bras de cliquet (42) variant sur sa longueur dans la direction longitudinale.

11. Mécanisme limiteur selon l'une quelconque des revendications 6 à 10, comprenant en outre un ressort spiral plat à enroulement inverse (100) en tant que réservoir de puissance, ayant une première extrémité attachée à une première bobine (101) et une deuxième extrémité attachée à une deuxième bobine (102), l'une des bobines (102) étant accouplée à l'organe d'entraînement (70).

12. Mécanisme limiteur selon l'une quelconque des revendications précédentes, dans lequel un élément supplémentaire (130) est pourvu de marquages visibles depuis l'extérieur du boîtier (10, 30, 40 ; 40'), prévus pour une indication visuelle de la dose réglée.

13. Dispositif d'injection comprenant un mécanisme limiteur selon l'une quelconque des revendications précédentes et une cartouche (20) contenant un médicament.
